# EUROPEAN PATENT APPLICATION

(11) **EP 4 585 220 A1**
(43) Date of publication of application: **16.07.2025**
(21) Application number: 23863505.6
(22) Date of filing: 06.09.2023
(51) Int. Cl.: A61K 35/76, A61K 38/19, A61K 38/17, A61K 9/00, A61P 35/00

(54) **NOVEL USE OF ONCOLYTIC VIRUS**

(30) Priority: 07.09.2022 KR 20220113801
(71) Applicant: SillaJen, Inc., Seoul 04525 (KR)
(72) Inventor: OH, Keunhee, Seongnam-si Gyeonggi-do 13615 (KR); LEE, Namhee, Seongnam-si Gyeonggi-do 13555 (KR); PARK, Mi Ju, Siheung-si Gyeonggi-do 15012 (KR); HONG, Yun Kyoung, Yangsan-si Gyeongsangnam-do 50654 (KR)
(74) Representative: J A Kemp LLP
(86) International application number: PCT/KR2023/013363
(87) International publication number: WO 2024/054040

(57) **Abstract**

The present invention relates to novel use of an oncolytic virus, and relates to an intravenously injectable composition for repeated administration, comprising, as an active ingredient, an oncolytic virus in which expression of a thymidine kinase (TK) gene is suppressed and genes encoding complement regulatory protein CD55 and granulocyte-macrophage colony-stimulating factor (GM-CSF) are inserted. In detail, the present invention relates to an intravenously injectable composition for repeated administration, comprising, as an active ingredient, an oncolytic virus in which: expression of a TK gene is suppressed; and a gene encoding CD55 fused with a transmembrane domain region of viral membrane protein to enable the expression of the complement regulatory protein CD55 in an intracellular mature virion (IMV) membrane and a gene encoding the GM-CSF are inserted. Once administered into the human body, the composition of the present invention, due to the expression of CD55 fused with the viral membrane protein H3 in the virus IMV membrane, avoids a neutralization reaction caused by antibodies, thereby enabling repeated intravenous administration of the oncolytic virus. In this regard, a reduction in anticancer efficacy by neutralizing antibodies can be prevented, and anticancer effects can be maximized.

## Description

### [Technical Field]

The present disclosure relates to novel use of an oncolytic virus, and relates to an intravenously injectable composition for repeated administration, comprising, as an active ingredient, an oncolytic virus in which expression of a thymidine kinase (TK) gene is suppressed and genes encoding complement regulatory protein CD55 and granulocyte-macrophage colony-stimulating factor (GM-CSF) are inserted. More particularly, the present disclosure relates to an intravenously injectable composition for repeated administration, comprising, as an active ingredient, an oncolytic virus in which: expression of a TK gene is suppressed; and a gene encoding complement regulatory protein CD55 fused with a transmembrane domain region of viral membrane protein to enable expression of CD55 in an intracellular mature virion (IMV) membrane and a gene encoding the GM-CSF are inserted.

### [Background Art]

Cancer is a leading cause of death worldwide, and with its enormous personal and societal burden, the importance of developing innovative anticancer treatments has gradually increased. Accordingly, various approaches based on advances in molecular biology have been attempted.

Oncolytic viruses, which have recently been in the spotlight as anticancer treatments, are replicable and infectious viruses. They are used to treat cancer by inserting specific genes that target genetically abnormal parts in tumor cells into wild-type or attenuated viruses. These genetically engineered oncolytic viruses spread through tissues with cell lysis after viral replication, and their spread occurs selectively in cancer cells and blood vessels surrounding the cancer cells. A representative oncolytic virus is lmlygic (talimogene laherparepvec) from Amgen in the United States, which has been approved by the U.S. Food and Drug Administration (FDA) as a treatment for melanoma in October 2015.

Meanwhile, intravenous injection of medicines is a convenient method for the rapid systemic delivery of drugs when oral administration is not possible, and it is simple to administer, making it one of the most preferred routes of drug delivery. In addition, intravenous injection allows the drug to reach the tumor through the blood vessels, and thus can deliver the drug in a consistent distribution within the tumor, allowing the efficacy of the drug to be evenly distributed throughout the tumor. However, since oncolytic virus preparations are quickly eliminated by the body's immune system such as the complement system and neutralizing antibodies upon intravenous injection. As a result, only a small fraction of the administered oncolytic virus reaches the tumor, limiting its therapeutic efficacy. Therefore, to increase the probability of the virus reaching the tumor, the oncolytic virus preparations are commonly injected directly into the target tumor rather than intravenously.

Intratumoral injection may generally be effectively applied in superficial cancers that are easily accessible, such as melanoma, breast cancer, and head and neck cancer. However, it is difficult to administer an effective dose to all tumor tissues in cases where there are multiple tumors in a single organ, deep solid tumors that have developed in areas that are not easily accessible, and cancers that have metastasized to multiple organs. In addition, intratumoral injection is a highly invasive procedure, making it less suitable for repeated administration compared to intravenous injection.

Despite the advantages of intravenous injection, the administration of oncolytic viruses via this route is challenging because foreign substances, such as viruses introduced into the bloodstream, are gradually eliminated by the body's defense mechanisms, thereby reducing the activity of the oncolytic virus. In other words, when a virus enters the body through the blood vessels, it activates the complement system present in the blood, and the activated complement either directly neutralizes the virus or facilitates the phagocytosis by macrophages to eliminate the virus.

In addition, when an oncolytic virus is administered to the body for treatment, neutralizing antibodies against the virus are generated by an acquired immune response. With repeated administrations, the production of antibodies becomes more robust and occurs in larger quantities. The generated neutralizing antibodies bind to surface antigens of the virus and prevent virus infection, resulting in a gradual decrease in the anticancer efficacy of the virus with repeated administration, and ultimately loss of anticancer efficacy. Consequently, there is a need for research and development focused on the creation of oncolytic viruses that can evade neutralization by antibodies, thereby enabling intravenous injection and repeated administration.

### [Disclosure]

### [Technical Problem]

An object of the present disclosure is to provide novel use of an oncolytic virus, and to provide an intravenously injectable composition for repeated administration, comprising, as an active ingredient, an oncolytic virus in which expression of a thymidine kinase (TK) gene is suppressed and genes encoding complement regulatory protein CD55 and granulocyte-macrophage colony-stimulating factor (GM-CSF) are inserted. More particularly, an object of the present disclosure is to provide an intravenously injectable composition for repeated administration, comprising, as an active ingredient, an oncolytic virus in which: expression of a TK gene is suppressed; and a gene encoding complement regulatory protein CD55 fused with a transmembrane domain region of viral membrane protein to enable the expression of CD55 in an intracellular mature virion (IMV) membrane and a gene encoding the GM-CSF are inserted.

The technical objectives to be achieved according to the technical idea of the invention disclosed in the present the specification are not limited to the objectives for solving the problems mentioned above, and other objectives not mentioned may also be clearly understood by those skilled in the art from the following description.

### [Technical Solution]

This will be specifically described as follows. Meanwhile, each of the descriptions and embodiments disclosed in the present application may also be applied to each other descriptions and embodiments. That is, all combinations of various elements disclosed in the present application fall within the scope of the present application. In addition, the scope of the present application cannot be considered limited by the specific descriptions set forth below.

In one aspect to achieve the above object, the present disclosure provides an intravenously injectable composition for repeated administration (or multiple administration), comprising, as an active ingredient, an oncolytic virus in which expression of a thymidine kinase (TK) gene is suppressed and genes encoding complement regulatory protein CD55 and granulocyte-macrophage colony-stimulating factor (GM-CSF) are inserted.

More specifically, the present disclosure provides an intravenously injectable composition for repeated administration, comprising, as an active ingredient, an oncolytic virus in which: expression of a TK gene is suppressed; and a gene encoding complement regulatory protein CD55 fused with a transmembrane domain region of viral membrane protein to enable the expression of CD55in an intracellular mature virion (IMV) membrane and a gene encoding the GM-CSF are inserted.

In the present disclosure, in order to protect the oncolytic virus from complement attack upon intravenous injection of the oncolytic virus, a complement regulatory protein, for example, CD55, which has the highest complement regulatory activity, was expressed on the IMV membrane to evade complement attack. In particular, the present inventors have conducted research efforts to develop an oncolytic virus capable of repeated intravenous administration, and discovered and demonstrated that the above-mentioned oncolytic virus evades neutralization by a viral antibody, thereby completing the present disclosure.

As used herein, the term "oncolytic vaccinia virus" may be referred to as an "oncolytic virus," which includes a "recombinant virus" engineered by the deletion of all or part of an endogenous gene, or the introduction of a foreign gene. Such an oncolytic virus may be a vaccinia virus, an adenovirus, a herpes simplex virus, a retrovirus, a reovirus, a Newcastle disease virus, a Coxsackie virus, an enterovirus, or a herpesvirus, etc.

In the present disclosure, the vaccinia virus may be, for example, but is not limited to, Western Reserve (WR), New York Vaccinia Virus (NYVAC), Wyeth, LC16m8, Lister, Copenhagen, Tian Tan, USSR, TashKent, Evans, International Health Division-J (IHD-J), or International Health Division-White (IHD-W) strain.

As used herein, the term "thymidine kinase (TK)" is an enzyme involved in the biosynthesis of nucleotides. The thymidine kinase (TK) encoded by the TK gene serves to bind phosphate at gamma (γ) position of ATP to thymidine to produce nucleotides that make up viral DNA. The TK may be, but is not limited to, a sequence such as GenBank: AAR17937.1 or AY313847.1. Specifically, the TK or its gene may be comprised of an amino acid sequence of GenBank: AAR17937.1 or a nucleotide sequence of GenBank: AY313847.1, but is not limited thereto. In addition, the TK or its gene may have a homology of about 90%, 91%, 92%, 93%, 94%, 95%, 96%, 97%, 98%, or 99% or more to the amino acid sequence of GenBank: AAR17937.1 or the nucleotide sequence of GenBank: AY313847.1.

As used herein, the term "granulocyte-macrophage colony-stimulating factor (GM-CSF)" is a protein that plays a pivotal role in the production, differentiation, and activation of various immune cells, including cells that become granulocytes, macrophages, and platelets. It is a cytokine belonging to a class of drugs called hematopoiesis (blood-forming agents), and is also known as sargramostim. GM-CSF is produced by many different cell types (e.g., activated T cells, B cells, macrophages, mast cells, endothelial cells, and fibroblasts) in response to cytokines or immune and inflammatory stimuli. In addition to granulocyte-macrophage progenitors, GM-CSF is also a growth factor for erythrocyte, megakaryocyte, and eosinophil progenitors. For mature hematopoietic cells, GM-CSF is a survival factor for granulocytes, monocytes/macrophages, and eosinophils, and activates their effector functions. GM-CSF has also been reported to have a functional role for non-hematopoietic cells. It may induce human endothelial cells to migrate and proliferate.

The GM-CSF may be, but is not limited to, the sequence of GenBank: M10663.1 or GenBank: X02333.1. Specifically, the GM-CSF gene may be composed of a nucleotide sequence of GenBank: M10663.1 or GenBank: X02333.1 or may be part of the sequence. The gene of the GM-CSF may have a homology of about 90%, 91%, 92%, 93%, 94%, 95%, 96%, 97%, 98%, or 99% or more to a nucleotide sequence of GenBank: M10663.1 or GenBank: X02333.1. In addition, the GM-CSF may be composed of an amino acid sequence of SEQ ID NO: 3 or 8 or may be part of the sequence, and may be composed of a nucleotide sequence of SEQ ID NO: 4 or 9 or may be part of the sequence.

As used herein, the term "complement regulatory protein" is a protein that precisely regulates a complement activation pathway *in vivo.* It is broadly divided into a serotype (soluble) regulatory protein group and a membrane-bound regulatory protein group. The serotype regulatory protein, for example, includes C4b-binding protein, H factor, SGP120, and properdin (P), etc. The membrane-bound regulatory protein, for example, includes CRI (CD35), CR2 (CD21), CR3 (CD11b/CD18), CR4 (CD11c/CD18), DAF (CD55), membrane cofactor protein (MCP, CD46), and CD59. Of these, only P acts to enhance complement activity, while the others act to attenuate complement activity. The complement regulatory protein contained in the recombinant vaccinia virus of the present disclosure may be, but is not limited to, CD35, CD21, CD18, CD55, CD46, or CD59, and specifically CD55. Specifically, the gene of the CD55 may be comprised of a nucleotide sequence of Genbank: NM_000574.3 or may be part of the sequence. The gene of the CD55 may have a homology of about 90%, 91%, 92%, 93%, 94%, 95%, 96%, 97%, 98%, or 99% or more to the nucleotide sequence of NCBI Reference Sequence: NM_000574.3. More specifically, the CD55 may be comprised of an amino acid sequence of SEQ ID NO: 1 or a nucleotide sequence of SEQ ID NO: 2.

As used herein, the term "transmembrane domain" also called a transmembrane region or a transmembrane segment, refers to a region of a membrane protein that spans across a lipid bilayer.

Specifically, the membrane protein of a vaccinia virus may be D8L, A16L, F9L, G9R, H3L, L1R, A9L, A13L, A21L, A28L, E10R, G3L, H2R, I2L, J5L, L5R, or O3L, and may comprise all or part of the membrane protein sequences, as shown in Table 1 below as an example.

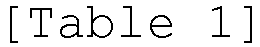

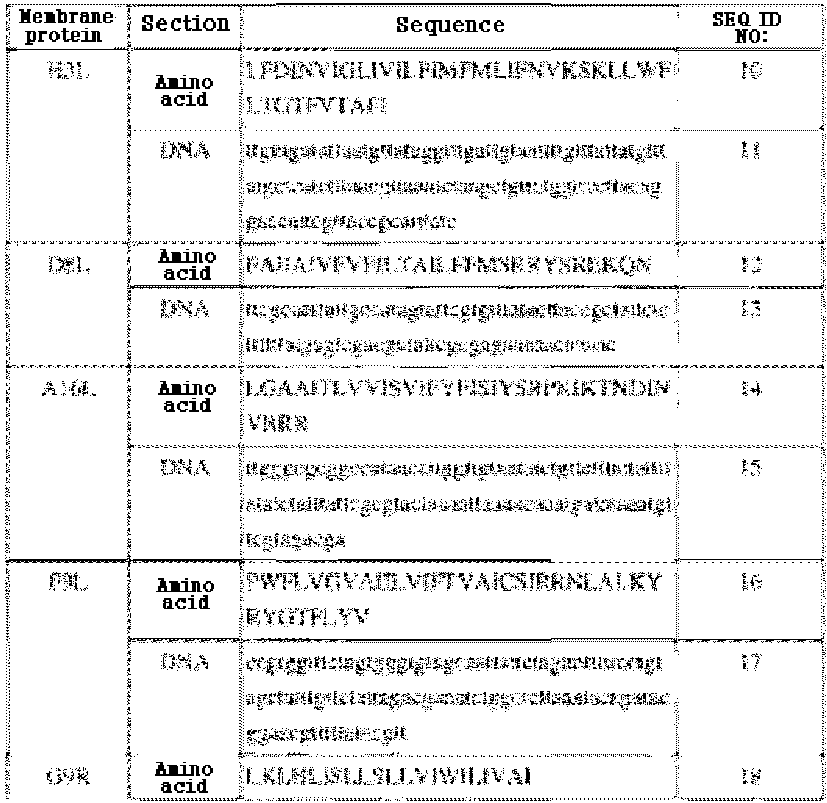

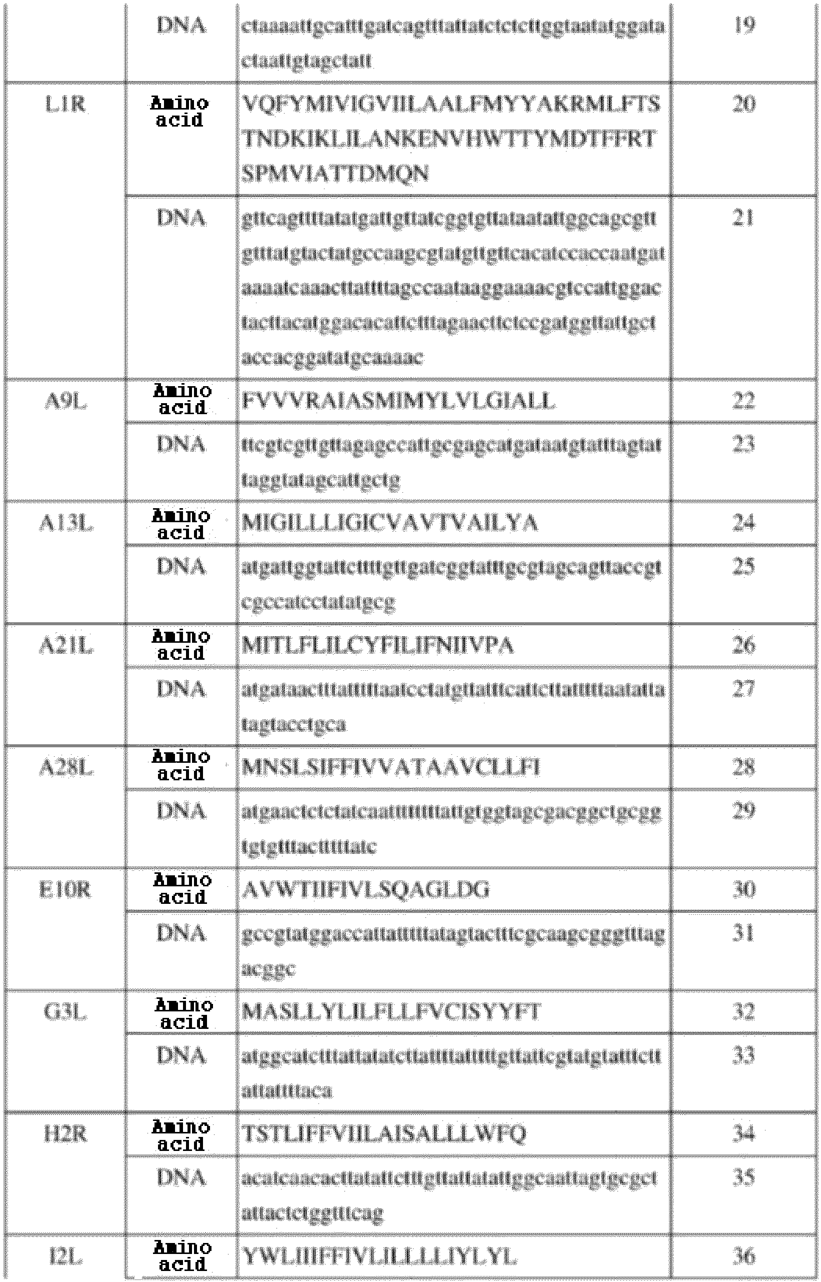

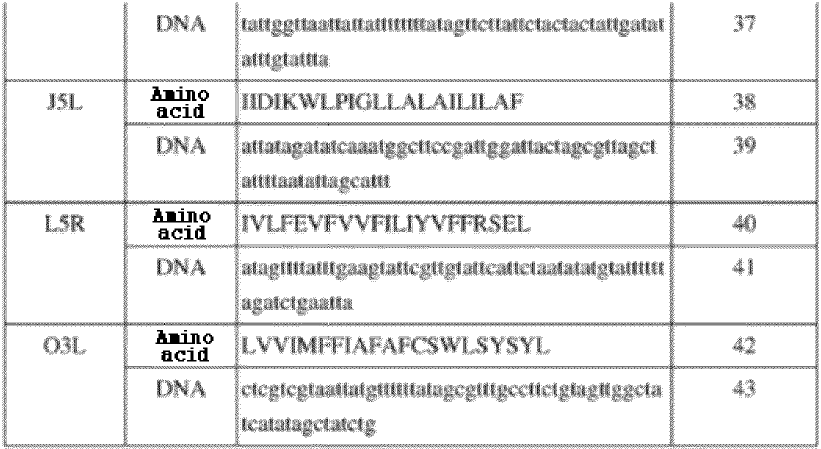

Here, the transmembrane domain of the complement regulatory protein and the oncolytic virus protein may be designed to include all or part of the genes through a genetic engineering process. Specifically, among the oncolytic viruses, the transmembrane domain of the vaccinia viral membrane protein may be composed of, but is not limited to, an amino acid sequence of SEQ ID NO: 10 or a nucleotide sequence of SEQ ID NO: 11.

Gene expression inhibition or gene inactivation according to the present disclosure means that a gene is not expressed, or only a part of the gene is expressed, due to deletion of part or all of the genes, or insertion of foreign genes into the gene, resulting in no activity of the protein encoded by the gene. The method of deleting the genes or inserting the foreign genes may be performed by methods well known in the art. For example, it may be performed by a method of inserting foreign genes disclosed in Molecular Cloning, A Laboratory Manual, Second Edition, by J. Sambrook, E. F. Fritsch and T. Maniatis (2003), Cold Spring Harbor Laboratory Press, Virology Methods Manual, edited by Brian W J Mahy and Hiliar O Kangro (1996) Academic Press and Expression of genes by Vaccinia virus vectors. This can be done by inserting a foreign gene as disclosed in Current Protocols in Molecular Biology, published by John Wiley and Son (1998), Chapter 16. Specifically, the suppression of the expression of the thymidine kinase gene may be caused by insertion of a foreign gene into part or all of the J2R region of the thymidine kinase. More specifically, in one embodiment of the present disclosure, the foreign genes were inserted using a T-Blunt^{™} PCR Cloning Kit (Solgent, Korea Cat No. SOT01-K020). The GM-CSF and complement regulatory protein contained in the oncolytic virus of the present disclosure may be expressed under the control of, but are not limited to, a late-early VACV p7.5 promoter, a vaccinia synthetic early-late promoter (pSEL), a vaccinia synthetic late promoter (pSL), a vaccinia modified H5 (mH5) promoter, a vaccinia short synthetic early-late pS promoter, a pLate promoter, a pC11R promoter, a pF11L promoter, a psFJ1-10 synthetic early promoter, a pHyb synthetic early promoter, any natural vaccinia early promoter, or a late-early optimized (LEO) promoter, respectively. As an example, the GM-CSF may be expressed under the control of the pSEL promoter, and the complement regulatory protein or CD55 may be expressed under the control of the pLate promoter. More specifically, the GM-CSF may be expressed under the control of the pSEL promoter of SEQ ID NO: 5 or the p7.5 promoter of SEQ ID NO: 7, and the complement regulatory protein may be expressed under the control of the pLate promoter of SEQ ID NO: 6.

The oncolytic virus of the present disclosure may further comprise genes that may increase the efficacy of cancer treatment. It is not limited to these genes. The oncolytic virus may comprise, for example, anticancer therapeutic genes, various immunomodulatory factors, intratumoral fibers, extracellular matrix degrading enzymes etc. For examples, it may further comprise, but is not limited to, IL-1, IL-2, IL-3, IL-4, IL-5, IL-6, IL-7, IL-8, IL-9, IL-10, IL-12, IL-13, IL-14, IL-15, IL-17, IL-18, IL-21, IL-23, IL-24, interferon-α, interferon-β, interferon-γ, CCL3, CCL5, CXCR4, CXCL9, CXCL10, CXCL11, interleukin superagonist (IL-2 superagonist, IL-15 superagonist), TGF-β blockade, TLR-2 agonist, TLR-3 agonist, TLR-7 agonist, STAT-3 inhibitor, PTENα, p53, p63, p73, adenosine deaminase-2, cancer-specific antigen, cancer-associated antigen, hyaluronidase, collagenase, protease, etc.

The composition of the present disclosure may be administered in combination with a substance that promotes immune activity so as to increase the efficacy of cancer treatment. For example, it may be used in combination with, but is not limited to, an immune checkpoint inhibitor, an anti-programmed cell death protein-1 (anti-PD-1) antibody, an anti-PD-L1 antibody, an anti-cytotoxic T lymphocyte antigen-4 (anti-CTLA-4) antibody, an anti-lymphocyte activation gene-3 (anti-LAG-3) antibody, an anti-T-cell membrane protein-3 (anti-TIM-3) antibody, an anti-T cell immunoreceptor with Ig and ITIM domains (anti-TIGIT) antibody, an anti-V-domain immunoglobulin-containing suppressor of T-cell activation (anti-VISTA) antibody, an Indoleamine 2,3-dioxygenase (IDO) inhibitor, a B and T lymphocyte attenuator (BLTA) inhibitor, a B7-H3 inhibitor, a B7-H4 inhibitor, a B7-H3 inhibitor, a galectin 9 (GAL9) inhibitor, a killer-cell immunoglobulin-like receptor inhibitor, chimeric antigen receptor T cells (CAR-T cells), chimeric antigen receptor natural killer cells (CAR-NK cells), or a combination of two or more of these.

Unlike other oncolytic viruses that are mainly administered intratumorally, the oncolytic virus of the present disclosure may be administered intravenously, and thus may be easily administered by a single route when combined with an immune checkpoint inhibitor, which is an intravenous preparation. In addition, since the oncolytic virus of the present disclosure does not develop drug resistance with reduced efficacy due to neutralization even when repeatedly administrated, a certain synergy effect of drug combination may be maintained even when repeatedly administered in combination with other anticancer drugs.

The composition of the present disclosure for the combination administration may be formulated for parenteral administration, but is not limited thereto. Specifically, the above parenteral administration may include intratumoral, intravenous, intra-arterial, hepatic arterial, intracerebral, intraperitoneal, intramuscular, intrasternal, subcutaneous, or intranasal administration, and may include, in one example, intratumoral, intravenous, intra-arterial, or intraperitoneal administration, and more specifically, may include intravenous administration.

The oncolytic virus of the present disclosure may preferably have a part or all of the K2L region encoding a serine protease inhibitor-3 (SPI-3) additionally deleted to inhibit the expression of a serine protease inhibitor-3. The K2 protein plays a role in inhibiting cell-to-cell fusion caused by vaccinia virus infection, and deletion of the K2L gene may lead to the formation of syncytia, resulting in rapid death of infected cells, particularly infected cancer cells.

In the present disclosure, the composition may preferably evade neutralization by antibodies against the virus.

In the present disclosure, the composition may preferably be for administration to a subject who possesses a neutralizing antibody to the oncolytic virus of the present disclosure. Such a subject may be a subject who has previously been administered the oncolytic virus of the present disclosure, or who has been infected with a smallpox virus or monkeypox virus, which are of the same family as the vaccinia virus of the present disclosure, or who has acquired a neutralizing antibody through vaccination, etc.

As used herein, the term "neutralizing antibody" refers to an antibody that protects cells by neutralizing the biological effects of infectious particles such as viruses when they enter the body. The neutralizing antibody generally performs an immune function that protects cells by binding to pathogens that are toxic to the body and inhibiting their activity, but it also has the same effect on oncolytic viruses injected for anticancer treatment, thereby reducing the anticancer efficacy caused by the oncolytic virus. Therefore, there is a problem that the number of injections is limited due to the formation of neutralizing antibodies against the virus, when the oncolytic virus is repeatedly injected.

As used herein, the term "neutralization" refers to an immunological detection ability of a specific antibody that prevents viruses, etc. from invading target cells. The neutralization of the antibody refers to the binding of neutralizing antibodies to a virus, which acts to wrap around an antigenic protein portion of the virus to convert it from an infectious state to a non-infectious state and prevent it from binding to the host's receptors, thus preventing it from causing infection.

In the present disclosure, the neutralization evasion may be due to fusing CD55 with the transmembrane domain of the vaccinia viral membrane protein H3 and expressing it on the membrane of IMV. Specifically, by expressing CD55 on the membrane of IMV using H3, the major antigen of the vaccinia virus, the exposure of the virus to the major antigen mya be limited, thereby evading the neutralization by antibodies against the virus.

Repeated administration of the oncolytic virus is possible by avoiding the neutralization by CD55, and thus it may exhibit significantly superior anticancer efficacy compared to a control virus that does not express CD55. In addition, an oncolytic virus in which K2L is deleted and CD55 is expressed, may exhibit significantly superior anticancer efficacy compared to a control virus.

Specifically, it was confirmed that in the case of the recombinant vaccinia virus expressing CD55 of the present disclosure, even if antibodies are formed by repeated intravenous administration of the virus, it was possible to avoid the neutralization by the viral antibody and exhibit anticancer efficacy, and experimentally, anticancer efficacy may be exhibited without being affected by neutralizing antibodies even when recombinant vaccinia virus is repeatedly administered intravenously after pre-injection of neutralizing antibodies against vaccinia virus or after induction of antibody formation against the virus by injection of vaccinia virus.

The intravenously injectable composition for repeated administration of the present disclosure may be provided as a pharmaceutical composition in one embodiment.

The specific dosage of the pharmaceutical composition of the present disclosure may be selected in various ways by those skilled in the art depending on factors such as the method of formulation, the condition and weight of the patient, the sex and age of the patient, the disease severity, the drug form, the route and duration of administration, rate of excretion, response sensitivity, etc., and the dosage and frequency are not intended to limit the scope of the present disclosure in any way. Specifically, the pharmaceutical composition of the present disclosure may comprise, but is not limited to, from 10⁵ to about 10¹³ plaque forming unit (pfu) of an oncolytic virus.

The pharmaceutical composition of the present disclosure may be administered to a patient at a dose of 1 x 10⁵ plaque forming unit (pfu) to 1 x 10¹³ pfu per single dose, and may be administered to a patient at a single dose of 1 x 10⁵ plaque forming unit (pfu) to 1 x 10¹³ pfu or multiple (i.e., 1 x 10⁸ pfu, 5 x 10⁸ pfu, 1 x 10⁹ pfu, etc.) doses.

Specifically, it may be administrated in the rage of 1 x 10⁵ pfu, 1 x 10⁶ pfu, 1 x 10⁷ pfu, 1 x 10⁸ pfu, 1 x 10⁹ pfu, 1 x 10¹⁰ pfu, 1 x 10¹¹ pfu, 1 x 10¹³ pfu or 1 x 10⁵ pfu to 1 x 10⁶ pfu, 1 x 10⁶ pfu to 1 x 10⁷ pfu, 1 x 10⁷ pfu to 1 x 10⁸ pfu, 1 x 10⁸ pfu to 1 x 10⁹ pfu, 1 x 10⁹ pfu to 1 x 10¹⁰ pfu, 1 x 10¹⁰ pfu to 1 x 10¹¹ pfu, 1 x 10¹¹ pfu to 1 x 10¹² pfu, or 1 x 10¹² pfu to 1 x 10¹³ pfu per single dose.

The pharmaceutical composition of the present disclosure may be administered as a single dose or as repeated (or multiple) doses (2, 3, 4, 5, 6, 7, 8, 9, 10, or more).

When the pharmaceutical composition of the present disclosure is administered twice or more, it may be administered 2 to 28 days after the first administration date or the immediately previous administration date. Specifically, it may be administered 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, or 28 days after the first administration day or the immediately previous administration day, but is not limited thereto.

When the pharmaceutical composition of the present disclosure is repeatedly administered, the administration interval may be daily, 2-day intervals, 3-day intervals, 4-day intervals, 5-day intervals, 6-day intervals, 1-week intervals, 2-week intervals, or 1-month intervals, or may be a regular interval of 1 day or more or a combination of several intervals, but is not limited thereto.

The pharmaceutical composition of the present disclosure may be prepared in the form of a pharmaceutical composition for the treatment or prevention of cancer, further comprising any appropriate carrier, excipient, or diluent conventionally used in the preparation of pharmaceutical compositions, wherein the carrier may comprise a non-naturally occurring carrier. Specifically, the pharmaceutical composition may be formulated and used in the form of oral formulations, such as powders, granules, tablets, capsules, suspensions, emulsions, syrups, aerosols, external preparations, suppositories, and sterile injection solutions, respectively, according to conventional methods. In the present disclosure, examples of carriers, excipients and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oils. When formulated, it is prepared using diluents or excipients such as fillers, bulking agents, binders, wetting agents, disintegrants, and surfactants that are commonly used. Solid preparations for oral administration include tablets, pills, powders, granules, capsules, etc., and these solid preparations are prepared by mixing at least one excipient, for example, starch, calcium carbonate, sucrose or lactose, gelatin, etc. Further, in addition to simple excipients, lubricants such as magnesium stearate and talc are also used. Liquid preparations for oral administration include suspensions, solutions, emulsions, syrups, etc., and may include a variety of excipients, for example, wetting agents, sweeteners, flavoring agents, preservatives, etc., in addition to water and liquid paraffin, which are simple diluents commonly used. Preparations for parenteral administration include sterile aqueous solutions, non-aqueous solutions, suspensions, emulsions, lyophilized preparations, and suppositories. As non-aqueous solvents and suspensions, propylene glycol, polyethylene glycol, vegetable oils, such as olive oil, and injectable esters, such as ethyl oleate, etc. may be used. As a base for the suppository, witepsol, macrogol, tween 61, cacao butter, laurin paper, glycerol gelatin, etc. may be used.

Specific formulations of pharmaceutical compositions are known in the art and reference can be made to, for example, Remington's Pharmaceutical Sciences (19th ed., 1995). The above reference is considered a part of the present specification.

The pharmaceutical composition of the present disclosure may be administered to mammals such as mouse, dog, cat, cow, horse, pig, a human, and preferably a human, via a variety of routes. As an example, the intravenously injectable composition for repeated administration of the present disclosure is resistant to the complement system of the human body and avoids the neutralization of antibodies against viruses upon intravenous injection, thereby allowing repeated administrations. Therefore, stable anticancer activity is maintained, so sufficient therapeutic efficacy may be exhibited even with intravenous administration.

In the present disclosure, in particular, the oncolytic virus of the present disclosure may be applied as a composition for repeated administration, for example, for multiple administration, such as two or more times, three or more times, and may be repeatedly injected intravenously by avoiding neutralization by antibodies against the virus formed in response to an immune response, and may therefore exhibit effective efficacy against deep-seated cancer or metastatic cancer occurring in inaccessible sites.

As used herein, the term "cancer (or tumor)" includes all cancers (or tumors), regardless of whether they are primary cancers or metastatic cancers. The cancer may include solid cancer or hematological cancer. The solid cancer may be any one selected from the group consisting of, but is not limited to, lung cancer, colorectal cancer, prostate cancer, thyroid cancer, breast cancer, brain cancer, head and neck cancer, esophageal cancer, skin cancer, thymic cancer, stomach cancer, colon cancer, liver cancer, ovarian cancer, uterine cancer, bladder cancer, rectal cancer, gallbladder cancer, bile duct cancer, pancreatic cancer, kidney cancer, osteosarcoma, sarcoma, chondrosarcoma, and combinations thereof. The hematological cancer may be any one selected from the group consisting of, but not limited to, lymphoma, leukemia, multiple myeloma, and combinations thereof.

As used herein, the term "metastatic cancer" refers to cancer that occurs when cancer cells leave the primary organ, spread to other organs, and proliferate. The metastatic cancer may include, but is not limited to, both the growth of cancerous tissue from the primary cancer that directly invades surrounding organs, and remote metastasis to another organ at a distance along a blood vessel or lymphatic vessel.

The present disclosure also relates to a method for treating cancer, comprising: intravenously administering an oncolytic virus of the present disclosure to a patient who has previously been administered the oncolytic virus of the present disclosure. Such an oncolytic virus may be administered to the patient in the form of the intravenously injectable composition described above.

The present disclosure also relates to a use for intravenously administering an oncolytic virus of the present disclosure to a patient who has previously been administered the oncolytic virus of the present disclosure. Such an oncolytic virus may be administered to the patient in the form of the intravenously injectable composition described above.

As used herein, the "patient who has previously been administered an oncolytic virus of the present disclosure" includes a patient who has been administered once, twice, three times, four times, five times, six times, seven times, or more, and refers to a subject who has a neutralizing antibody against an oncolytic virus of the present disclosure.

The present disclosure also relates to a composition for administration of the oncolytic virus of the present disclosure to a cancer patient who has been infected with a vaccinia virus or a pox virus of the same family (e.g., smallpox virus or monkeypox virus) or who has been vaccinated to prevent infection by the virus, based on the excellent neutralizing antibody evasion ability.

Specifically, when a person has been infected with a virus, or has been vaccinated, neutralizing antibodies against the virus in the body are acquired through active immunity. When the human body is exposed to the virus again, the neutralizing antibodies formed bind to the antigen of the virus and inhibit reinfection. When a cancer patient has been infected with a virus, or has been vaccinated, or has already been administered an oncolytic virus for therapeutic purposes, neutralizing antibodies against the corresponding oncolytic virus are formed, resulting in continuous decrease in anticancer efficacy upon repeated administration and ultimately loss of anticancer efficacy. The oncolytic vaccinia virus of the present disclosure avoids neutralization by viral antibodies, and may therefore be repeatedly administered intravenously without a decrease in anticancer efficacy, even in a cancer patient who has been infected with pox viruses such as smallpox or monkeypox virus, who has been vaccinated to prevent infection by these viruses, or who has previously been administered oncolytic virus.

The composition comprises the contents of an intravenously injectable composition for repeated administration as previously mentioned herein.

In addition, the present disclosure relates to a method for treating cancer or a use for treating cancer, comprising: administering an oncolytic virus of the present disclosure to a subject who has been infected with a vaccinia virus or a pox virus of the same series (e.g., smallpox virus or monkeypox virus) or who has been vaccinated.

In one aspect to achieve the above object, the present disclosure provides a method for preventing or treating cancer, comprising: administering to a subject an intravenously injectable composition for repeated administration, comprising, as an active ingredient, an oncolytic virus in which: expression of a TK gene is suppressed; and a gene encoding complement regulatory protein CD55 fused with a transmembrane domain region of viral membrane protein to express the CD55 in an intracellular mature virion (IMV) membrane and a gene encoding granulocyte-macrophage colony-stimulating factor (GM-CSF) are inserted.

As used herein, the term "composition" is as described above.

The individual may be a mammal, specifically, but is not limited to, a human, cow, sheep, goat, horse, pig, dog, cat, rabbit, rat, mouse, fish, bird, etc. In addition, the individual may be a subject who has been infected with a vaccinia virus or a pox virus of the same family (e.g., smallpox virus or monkeypox virus) or who has been vaccinated, but is not limited thereto.

In the present disclosure, the method of treating cancer includes a method of treating through combination administration with conventionally known anticancer agents and anticancer therapies.

Another aspect of the present disclosure to achieve the above objective provides a use of a composition comprising the oncolytic virus, for the prevention or treatment of cancer.

### [Advantageous Effects]

Once administered into the human body, the composition of the present disclosure enables repeated intravenous administration of an oncolytic virus by avoiding neutralization by antibodies against the virus through the expression of CD55. In this regard, a reduction in anticancer efficacy by neutralizing antibodies can be prevented, and anticancer effects can be maximized.

### [Description of Drawings]

FIG. 1 is a schematic diagram of a recombinant vaccinia virus SJ-607 in which the J2R gene is deleted and human GM-CSF and human CD55 fused with a transmembrane domain are introduced.
FIG. 2 is a schematic diagram of a recombinant vaccinia virus mSJ-612 which has deleted K2L gene and J2R gene and expresses mouse GM-CSF.
FIG. 3 is a schematic diagram of a recombinant vaccinia virus mSJ-650 which has deleted K2L gene and J2R gene and expresses mouse GM-CSF and human CD55 fused with a transmembrane domain.
FIG. 4 shows the results of confirming the neutralizing antibody titer over time when a recombinant vaccinia virus SJ-607 of the present disclosure was administered intravenously to BALB/c mice.
FIG. 5 shows the results of confirming the neutralizing antibody titer over time when recombinant vaccinia virus JX-594 as a control group was administered intravenously to BALB/c mice.
FIG. 6 shows the results of confirming the neutralizing antibody titer over time when a recombinant vaccinia virus SJ-607 of the present disclosure was administered intravenously to C57BL/6 mice.
FIG. 7 shows the results of confirming the neutralizing antibody titer over time when recombinant vaccinia virus JX-594 as a control group was administered intravenously to C57BL/6 mice.
FIG. 8 shows the results of confirming the amount of antibodies produced against vaccinia virus JX-594 when JX-594 and SJ-607 were repeatedly administered intravenously to BALB/c mice.
FIG. 9 shows the results of confirming the amount of antibodies produced against vaccinia virus SJ-607 when JX-594 and SJ-607 were repeatedly administered intravenously to BALB/c mice.
FIG. 10 shows the results of measuring the neutralizing antibody titer to JX-594 or SJ-607 of antibodies against vaccinia virus present in the blood when JX-594 and SJ-607 were repeatedly administered intravenously to BALB/c mice.
FIG. 11 shows the results of measuring the neutralizing antibody titer to JX-594 or SJ-607 of antibodies against vaccinia virus present in the blood when JX-594 and SJ-607 were repeatedly administered intravenously to C57BL/6 mice.
FIG. 12 shows the results of measuring the tumor growth inhibition efficacy when mSJ-650 expressing CD55 on the IMV membrane or mSJ-612 that does not express CD55 were repeatedly administered intravenously at low or high doses to a mouse breast cancer model.
FIG. 13 shows the results of measuring changes in body weight when mSJ-650 or mSJ-612 was repeatedly administered intravenously at low or high doses to a mouse breast cancer model.
FIG. 14 shows the results of measuring the neutralizing antibody titers against mSJ-650 or mSJ-612 of the antibodies formed in response to the virus in the blood, 3 weeks after the initial virus administration, following repeated intravenous administration of mSJ-650 or mSJ-612 in a mouse breast cancer model.
FIG. 15 shows the results of measuring the neutralizing antibody titers against mSJ-650 or mSJ-612 in the blood of mice, 4 weeks after the initial virus administration, following repeated intravenous administration of mSJ-650 to BALB/c to collect serum containing neutralizing antibodies against the oncolytic vaccinia virus.
FIG. 16 shows the results of measuring the tumor growth inhibition efficacy after repeated intravenous administration of mSJ-650 or mSJ-612, following the administration of serum containing neutralizing antibodies via the mouse tail vein every 1 day before each virus administration in a mouse breast cancer model.
FIG. 17 shows the results of measuring the body weight changes after repeated intravenous administration of mSJ-650 or mSJ-612, following the administration of serum containing neutralizing antibodies via the mouse tail vein every 1 day before each virus administration in a mouse breast cancer model.
FIG. 18 shows the results of measuring the tumor growth inhibition efficacy following repeated intravenous administration of mSJ-650 or mSJ-612, after pre-administering the virus to induce the formation of neutralizing antibodies against the vaccinia virus in individuals, in a mouse breast cancer model.
FIG. 19 shows the results of measuring the body weight changes following repeated intravenous administration of mSJ-650 or mSJ-612, after pre-administering the virus to induce the formation of neutralizing antibodies against the vaccinia virus in individuals, in a mouse breast cancer model.
FIG. 20 shows the results of measuring tumor size and weight 21 days after tumor transplantation, following pre-administration of the virus to induce the formation of neutralizing antibodies against the vaccinia virus in individuals, and the repeated intravenous administration of mSJ-650 or mSJ-612 in a mouse breast cancer model..

### [Best Mode]

Hereinafter, the present disclosure will be described in more detail with reference to the following Examples. However, these Examples are intended to illustrate the present disclosure by way of example, and the scope of the present disclosure is not limited to these Examples.

The present inventors constructed a vector in which: expression of a TK gene is suppressed; and a gene encoding complement regulatory protein CD55 fused with a transmembrane domain region of viral membrane protein to enable expression of CD55in an intracellular mature virion (IMV) membrane and a gene encoding granulocyte-macrophage colony-stimulating factor (GM-CSF) are inserted, and generated a recombinant vaccinia virus in which the expression of the genes was induced by homologous recombination between the vector and the vaccinia virus. They confirmed that when the recombinant vaccinia virus was administered in vivo, it avoided the neutralization caused by the virus, thereby enabling repeated administration.

### Preparation Example 1: Construction of recombinant vaccinia virus SJ-607 having deleted J2R gene and expressing human GM-CSF and human CD55

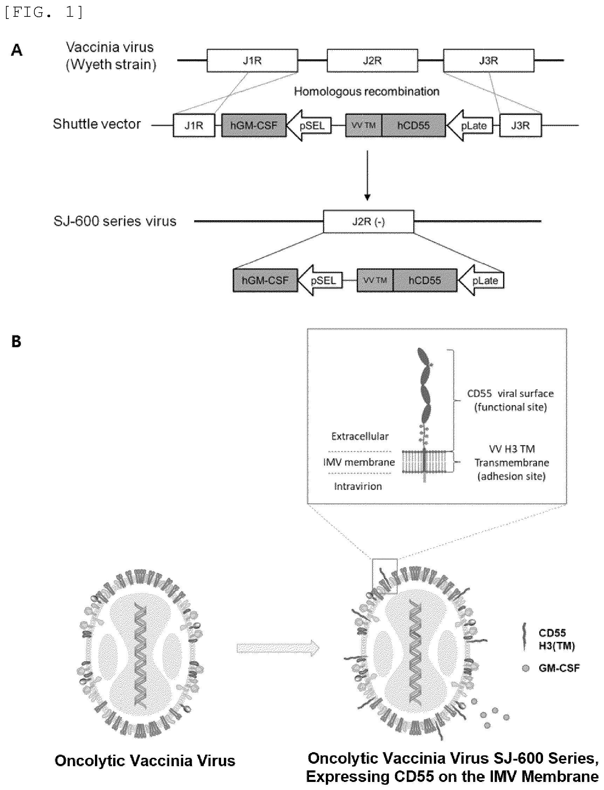

As shown in FIG. 1, a J2R region encoding thymidine kinase (TK) was completely deleted in the Wyeth stain of vaccinia virus, and genes encoding human CD55 (hCD55) and human GM-CSF (hGM-CSF) were inserted into this region to prepare a recombinant vaccinia virus. In addition, in order to express the hCD55 on the intracellular mature virus (IMV) membrane of the virus, the hCD55 gene, excluding the GPI anchor region, was fused with the transmembrane domain and intravirion region (C-terminal region) of the vaccinia viral membrane protein H3, and pLate was used as the promoter for expression. The specific process is as follows.

### 1.1 Construction of plasmid vectors in which expression of hGM-CSF and hCD55 genes was induced

In order to delete a J2R region of vaccinia virus, J1R and J3R genes, which are the left and right flanking regions of the J2R region, were cloned into a T-blunt^{™} vector (Solgent) using a NEBuilder^{®} HiFi DNA Assembly Cloning Kit (NEW ENGLAND BioLabs, Catalog No. M5520A). J1R and J3R DNA were amplified by PCR using the corresponding sites of JX-594 (Pexastimogene Devacirepvec, Pexa-Vec) as templates. The J1R is identical to the region of amino acids 1 to 154 of J1R (Protein id = AAR17936.1) of vaccinia virus Acambis2000 strain, except that amino acid 118 is substituted from Alanine to Serine. J3R (Protein id = AAR17938.1) is the region corresponding to amino acids 1 to 145. The shuttle vector was constructed by cloning the hGM-CSF (GenBank: M10663.1) expressed under the control of the vaccinia virus synthetic early-late promoter, the complement regulatory protein hCD55 gene (GenBank: NM_000574.3) expressed under the control of the vaccinia virus late promoter, and the transmembrane domain region and intravirion region of vaccinia virus H3L into this vector. The amino acid sequences and gene sequences of hCD55 and hGM-CSF used are shown in Table 2 as SEQ ID NOs: 1 to 4, respectively.

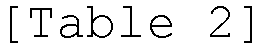

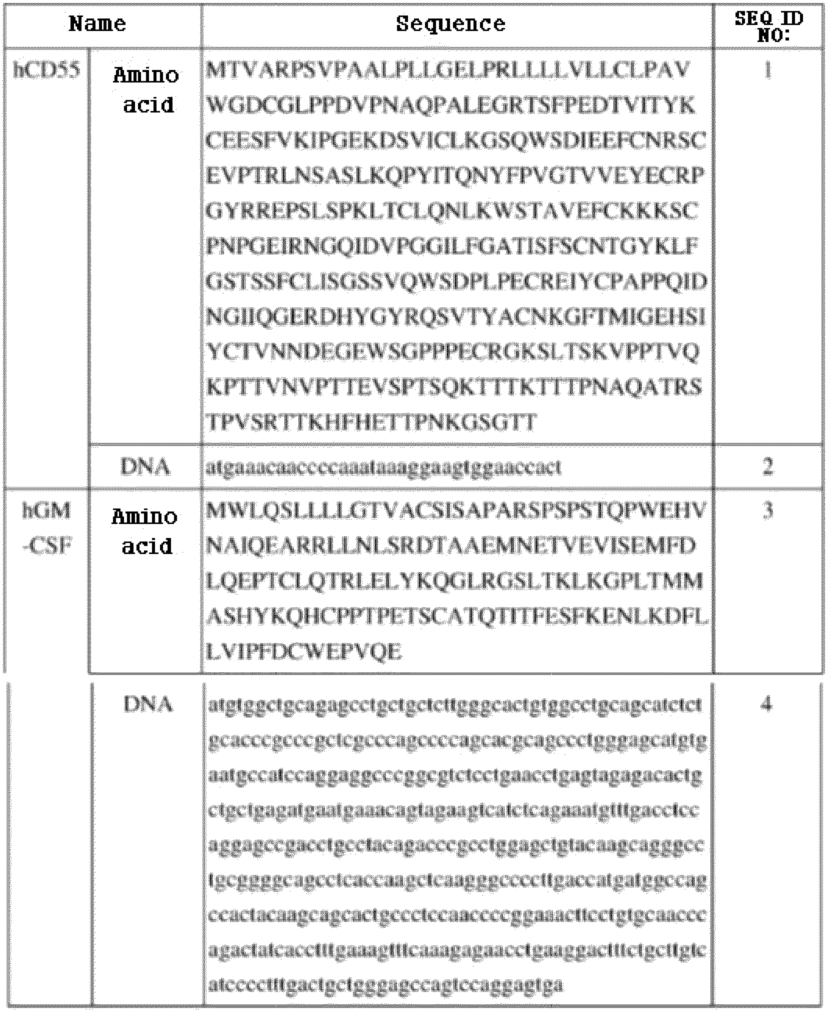

The hGM-CSF and the transmembrane domain and intravirion region of H3L were PCR amplified from JX-594. hCD55 was PCR amplified from a pCMV3 plasmid vector(Sinobio, Catalog No. HG10101-UT). hGM-CSF (GenBank: M10663.1) was the region of amino acids 1 to 145 and was expressed under the control of a synthetic early-late promoter (aaaaattgaaattttattttttttttttttggaatataaata, SEQ ID NO: 5, pSEL, PCR amplified from Pexa-Vec). hCD55 (GenBank: NM_000574.3) is the region of amino acids 1 to 352, with the GPI anchor region deleted, and the promoter for expression is a synthetic late promoter (tttttttttttttttttttttggcatataaata, SEQ ID NO: 6, pLate, synthesis request from Macrogen). The 3'end of the hCD55 gene was fused with the transmembrane domain and intravirion region of vaccinia virus H3L to allow CD55 to be expressed on the IMV membrane of the virus. Information and nucleotide sequences for the transmembrane domain region of H3L used are shown in Tables 3 and 4.

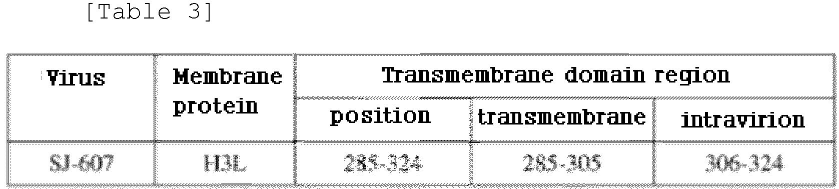

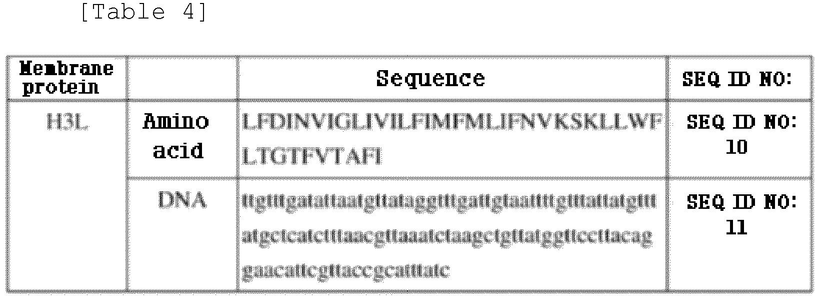

### 1.2. Construction of SJ-607 recombinant vaccinia virus by homologous recombination

The SJ-607 recombinant vaccinia virus was generated by homologous recombination of wild-type Wyeth strain of vaccinia virus and a plasmid vector encoding the hGM-CSF and hCD55 genes constructed previously.

The wild-type Wyeth strain was generated by inserting J2R gene from Western Reserve strain of vaccinia virus (ATCC, Catalogue No. VR-1354) into an inactivated J2R region (TK region) of JX-594 (Pexastimogene Devacirepvec, Pexa-Vec). The J2R of Western Reserve strain (Protein id = YP_232976.1) was the region of amino acids 1 to 178. The J2R gene of the Western Reserve strain was amplified by PCR, and the wild-type Wyeth strain was constructed by homologous recombination of this PCR product with JX-594.

In order to generate SJ-607, 143B osteosarcoma cells (Creative Bioarray) were infected with the wild-type Wyeth strain and transfected with plasmid vectors encoding hGM-CSF and hCD55. In order to select recombinant virus SJ-607 plaques with the TK region deleted, 143B osteosarcoma cell lysates infected with vaccinia virus and transfected with the plasmid vector were incubated with the TK deletion selection reagent 5-Bromo-2'-deoxyuridine (BrdU). The selected recombinant virus plaques were further passaged twice in 143B cells to obtain purified single clone SJ-607. The nucleotide sequence of the recombination site of SJ-607 was confirmed by sequencing.

Finally, the structure of SJ-607 generated in Preparation Example above is as shown in FIG. 1.

### Preparation Example 2: Construction of recombinant vaccinia virus mSJ-612 with deletion of K2L and J2R genes and expression of mouse GM-CSF

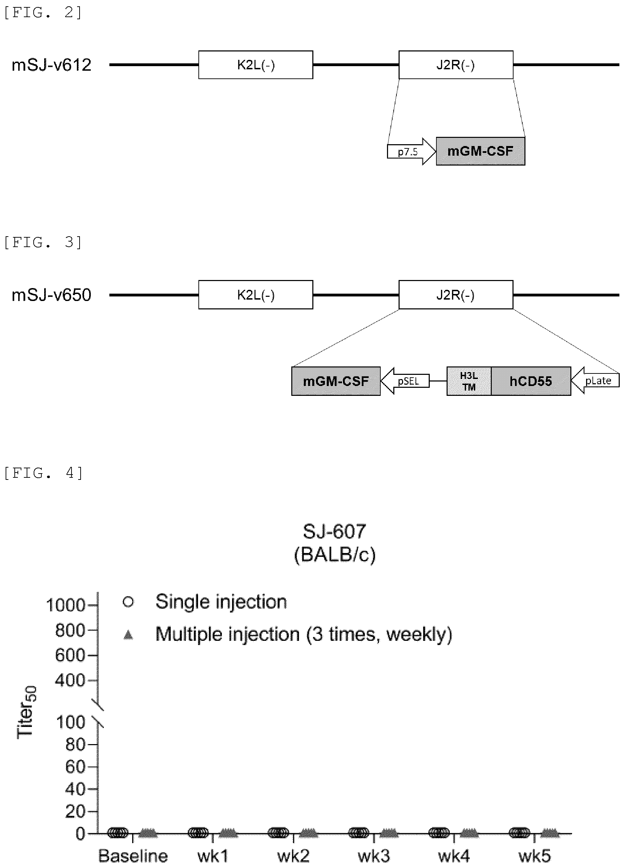

As shown in FIG. 2, a recombinant vaccinia virus was generated by inactivating TK expression through the insertion of mouse GM-CSF (mGM-CSF) into the J2R region, which encodes thyimidine kinase, and by deleting the K2L region, which encodes serine protease inhibitor-3 (SPI-3), from the Wyeth strain of vaccinia virus. The vaccinia virus promoter p7.5 (ccgtgcaataaattagaatatattttctacttttacgagaaattaattattgtattta ttatttatgggtgaaaaacttactataaaaagcgggtgggtttgga, SEQ ID NO: 7) was used as the promoter for mGM-CSF expression. The specific methods are as follows.

### 2.1. Construction of plasmid vector deleting K2L gene

In order to delete a K2L region of vaccinia virus, K1L and K3L to K4L genes, which are the left and right flanking regions of the K2L region, were cloned into a T-blunt^{™} vector. K1L and K3L to K4L DNA were amplified by PCR using the corresponding sites of JX-594 (Pexastimogene Devacirepvec, Pexa-Vec) as templates. The K1L is identical to the region of amino acids 1 to 154 of K1L (Protein id = AAR17875.1) of vaccinia virus Acambis2000 strain. K3L (Protein id = AAR17877.1) is identical to the region of amino acids 1 to 88, and K4L (Protein id = AAR17878.1) is identical to the region of amino acids 382 to 424.

### 2.2. Construction of mSJ-612 recombinant vaccinia virus by homologous recombination

The recombinant vaccinia virus mSJ-612 was generated by homologous recombination of a TK-expression-deactivated Wyeth strain of vaccinia virus (provided by Jennerex) through the insertion of mGM-CSF (GenBank: X02333.1) into the J2R region, using a plasmid vector previously constructed for the deletion of the K2L region. The amino acid sequence and gene sequence of the mouse GM-CSF used are shown in Table 5 below as SEQ ID NOs: 8 and 9, respectively.

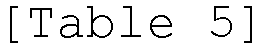

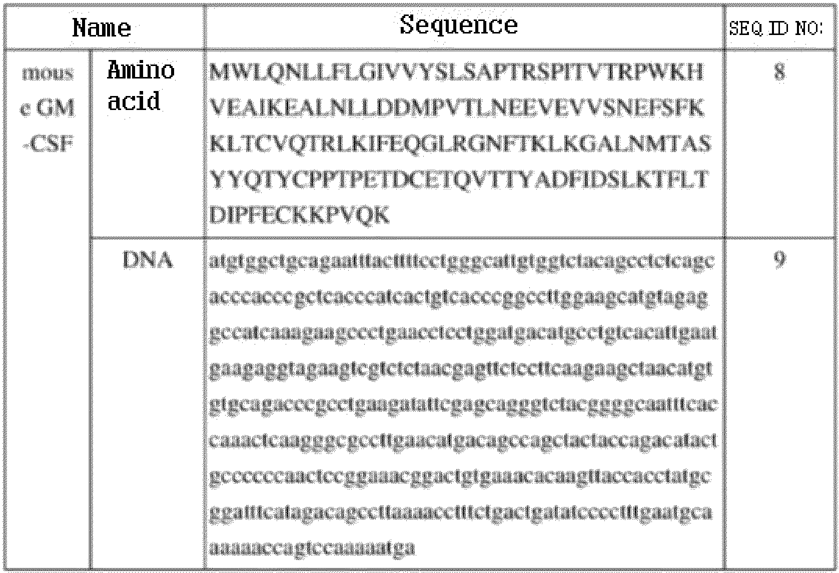

In order to generate mSJ-612, U-2 OS osteosarcoma cells (ATCC) were infected with the virus whose expression of TK was inactivated by inserting mGM-CSF into the J2R region, and transfected with a plasmid vector for deleting the K2L region. Subsequently, the recombinant virus mSJ-v612 in which the K2L region was deleted was prepared by a conventional method. The nucleotide sequence of the recombination site was confirmed by sequencing.

### Preparation Example 3: Construction of recombinant vaccinia virus mSJ-650 with deletion of K2L and J2R genes and expressing mouse GM-CSF and human CD55

First, the K2L region encoding a serine protease inhibitor-3 (SPI-3) of the Wyeth strain of vaccinia virus was deleted, and then mGM-CSF and hCD55 were inserted into the J2R region encoding thymidine kinase (TK) to generate a recombinant vaccinia virus mSJ-v650 (FIG. 3). In order to express the hCD55 on the IMV membrane of the virus, the hCD55 gene, excluding the GPI anchor region, was fused with the transmembrane domain and intravirion region of the vaccinia viral membrane protein H3. pLate was used as the promoter for expression. The specific methods are as follows.

### 3.1 Plasmid vector deleting K2L gene

The plasmid vector used in the preparation of the recombinant bacterial virus in Preparation Example 2 above was used.

### 3.2. Construction of plasmid vector with J2R gene deleted and encoding expression of mouse GM-CSF and human CD55 genes

A plasmid vector with J2R gene deleted and encoding mGM-CSF and hCD55 genes was constructed in the same manner as in Preparation Example 1 except for the use of mGM-CSF (GenBank: X02333.1) expressed under the control of vaccinia virus p7.5 promoter in the plasmid vector used for the construction of the recombinant vaccinia virus in Preparation Example 1 above.

### 3.3. Construction of mSJ-650 recombinant vaccinia virus by homologous recombination

The recombinant vaccinia virus mSJ-650 was generated by homologous recombination of a wild-type Wyeth strain of vaccinia virus with a plasmid vector previously constructed for the deletion of K2L region, followed by homologous recombination with a plasmid vector for the deletion of J2R gene and encoding the mGM-CSF and hCD55 genes.

First, a recombinant vaccinia virus with a K2L gene deletion was generated using the same method as in Preparation Example 2, in which a plasmid vector for the deletion of the K2L region, constructed in Preparation Example 2 was introduced into the wild-type Wyeth strain of vaccinia virus constructed in Preparation Example 1. Subsequently, recombinant vaccinia virus mSJ-650 with the deletion of the K2L and J2R genes and the expression of mGM-CSF and hCD55 was constructed, using the same method as in Preparation Example 1, employing a recombinant vaccinia virus with a K2L gene deletion and a plasmid vector for the deletion of J2R gene and encoding mGM-CSF and hCD55 genes. The nucleotide sequence of the mSJ-650 recombination site was confirmed by sequencing.

### Example 1: Confirmation of neutralizing antibody formation after administration of SJ-607 in mice

The recombinant vaccinia virus SJ-607 prepared in the Preparation Example 1 above was administered intravenously into the tail of BALB/c mice or C57BL/6 mice (5 x 10⁶ pfu, once or three times at 1-week intervals). JX-594 (Pexa-vec), which has inactivated TK and expresses hGM-CSF and Lac-Z, was used as a control virus. Blood samples were collected from the orbital vein of mice immediately before virus administration, and then were collected at 1-week intervals for 5 weeks (before virus administration, and 7, 14, 21, 28, and 35 days after virus administration, respectively). The collected blood samples were left at room temperature for 60 minutes, and then centrifuged at 4°C to isolate serum. The isolated serum was heat-treated at 56°C for 30 minutes to inactivate complement in the serum, and then the formation of neutralizing antibodies against JX-594 or SJ-607 was measured. The heat-treated serum was diluted 10- to 316,228-fold with cell culture medium and incubated with JX-594 or SJ-607 for 2 hours to allow the neutralizing antibodies against oncolytic vaccinia virus in the serum to neutralize the virus. U-2 OS human osteosarcoma cells seeded in a 96-well plate were infected with JX-594 or SJ-607 diluted with serum and incubated at 37° C for 3 days, and then the cell death effect was observed. The viability of U-2 OS cells 3 days after virus infection was evaluated by measuring the absorbance at 450 nm after treatment with a Cell Counting Kit-8 (Dojindo). Neutralizing antibody titer (Titer₅₀) was determined as the maximum dilution at which 50% or more of U-2 OS cells survived.

As a result, it was confirmed that in the case of BALB/c mice, when the oncolytic vaccinia virus SJ-607 was administered intravenously, no significant neutralization was observed against SJ-607 during all observation periods after administration, both in the single dose and in the three doses at 1-week intervals (FIG. 4).

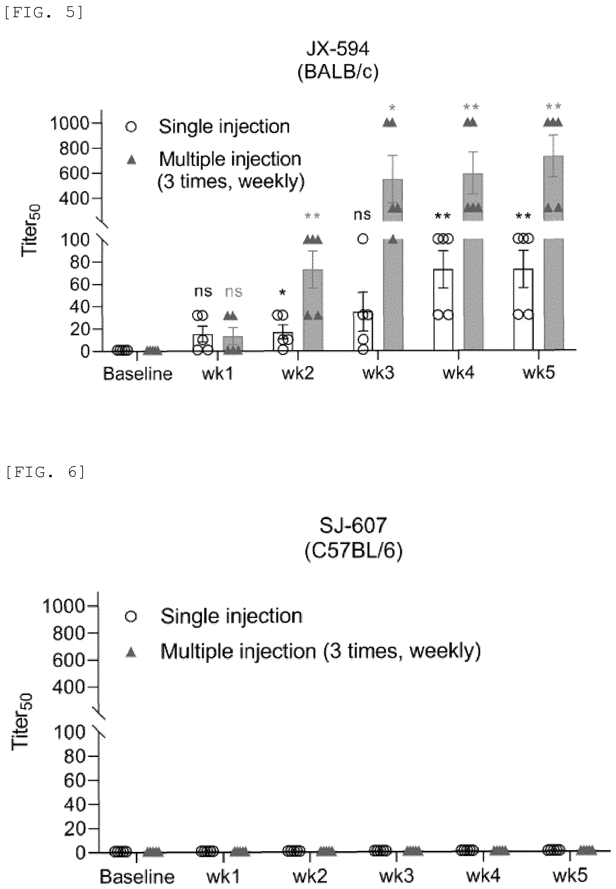

On the other hand, it was confirmed that when the oncolytic vaccinia virus JX-594 was administered intravenously, neutralizing antibodies began to form 2 weeks after administration, and the maximum value was measured at week 4 for single dose, and the maximum value was measured at week 3 for three doses at 1-week intervals, and the maximum value was maintained until the last observation time point, at week 5 after administration. In addition, the neutralizing efficacy was increased by 10-fold or more in the condition of the three doses at 1-week intervals compared to the single dose (FIG. 5).

In addition, it was confirmed that, in the case of C57BL/6 mice, when the oncolytic vaccinia virus SJ-607 was administered intravenously, no significant neutralization response to SJ-607 was observed in both single dose and three doses at 1-week intervals for up to 35 days after administration (FIG. 6).

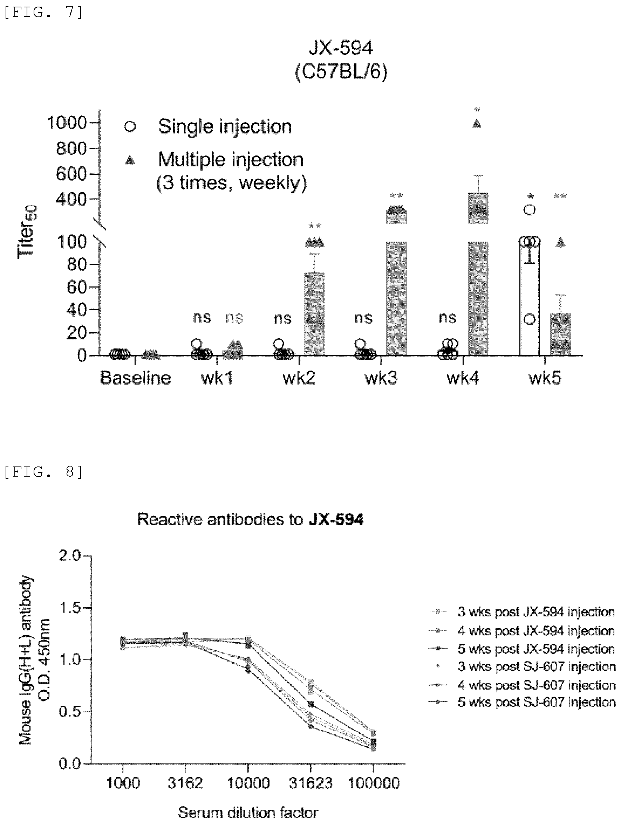

On the other hand, it was confirmed that when oncolytic vaccinia virus JX-594 was administered intravenously, neutralization efficacy was observed only at the last observation time point, i.e., at week 5 of administration for single dose, and the neutralization to JX-594 began to appear at week 2 of administration, and the maximum value was measured at week 4 for three doses at 1-week intervals (FIG. 7).

### Example 2: Confirmation of antibody formation against SJ-607 vaccinia virus

To confirm the formation of antibodies against vaccinia virus SJ-607, naive BALB/c mice were administered oncolytic vaccinia virus JX-594 or SJ-607 through the tail vein at 5 × 10⁶ pfu three times at 1-week intervals. At weeks 3, 4, and 5 after the initial virus administration, blood samples were collected from the orbital vein of the mice, left at room temperature for 60 minutes, and then centrifuged at 4°C to isolate serum. The isolated serum was heat-treated at 56°C for 30 minutes to inactivate complement in the serum. Antibodies against vaccinia virus present in serum were quantified by enzyme-linked immunosorbent assay (ELISA). Clear Flat-Bottom Immuno 96-well plates were treated with vaccinia virus JX-594 or SJ-607 and incubated overnight at 4°C to coat the viruses. The next day, after blocking with 5% BSA, 0.05% Tween 20 solution for 2 hours, heat-treated serum diluted 10- to 100,000-fold with PBS was reacted for 1 hour at room temperature. To detect antibodies binding to vaccinia virus in serum, anti-mouse secondary antibodies (Invitrogen, Catalog No. G21040) conjugated with HRP were reacted at room temperature for 30 minutes, and then the activity of HRP was analyzed by absorbance at 450 nm by treating TMB substrate.

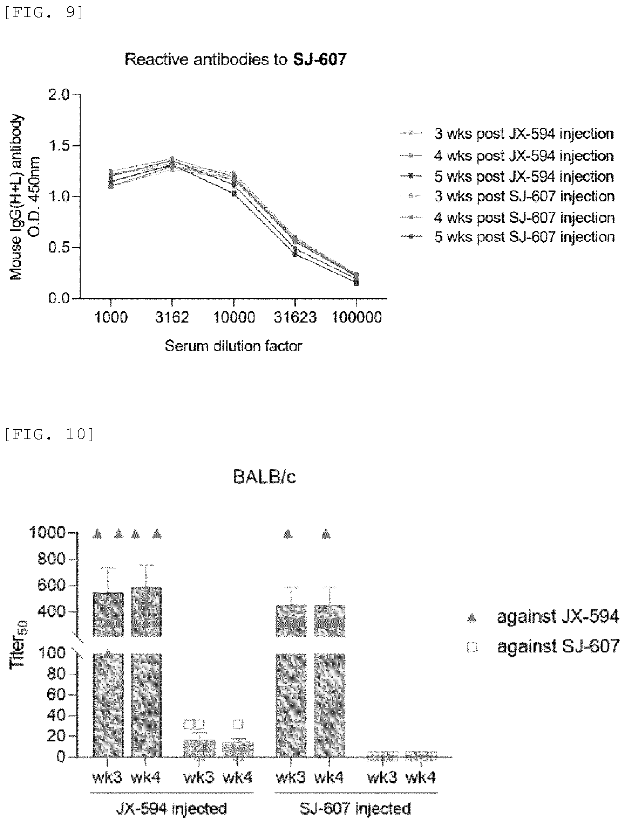

As a result, antibodies against vaccinia virus JX-594 were generated to the same extent in both mice repeatedly administered with vaccinia virus JX-594 and mice repeatedly administered with vaccinia virus SJ-607 (FIG. 8), and antibodies against vaccinia virus SJ-607 were generated to the same extent in both mice repeatedly administered with JX-594 and mice repeatedly administered with SJ-607 (FIG. 9). Therefore, it was confirmed that intravenous administration of oncolytic vaccinia virus induced antibody formation against vaccinia virus at a similar level regardless of hCD55 expression on the IMV membrane.

### Example 3: Confirmation of neutralization evasion ability of SJ-607 against vaccinia viral antibodies

To confirm the neutralization evasion ability of SJ-607 vaccinia virus against vaccinia virus-specific antibodies, naïve BALB/c or C57BL/6 mice were administered oncolytic vaccinia virus JX-594 or SJ-607 through the tail vein at 5 x 10⁶ pfu once or three times at 1-week intervals. At weeks 3 and 4 after the initial virus administration, blood samples were collected from the orbital vein of the mice, left at room temperature for 60 minutes, and then centrifuged at 4°C to isolate serum. The isolated serum was heat-treated at 56°C for 30 minutes to inactivate complement in the serum. The neutralization of vaccinia virus-specific antibodies against JX-594 and SJ-607 in the blood of mice that received repeated intravenous administration of JX-594, and the neutralization of vaccinia virus-specific antibodies against JX-594 and SJ-607 in the blood of mice that received repeated intravenous administration of SJ-607, were evaluated by a neutralizing antibody titer assay. The heat-treated serum was diluted 10- to 316,228-fold with cell culture medium and incubated with JX-594 or SJ-607 for 2 hours to allow the neutralizing antibodies against oncolytic vaccinia virus in the serum to neutralize the virus. U-2 OS human osteosarcoma cells seeded in 96-well plates were infected with JX-594 or SJ-607 diluted with serum and incubated at 37° C for 3 days, and then cell death effect was observed. The viability of U-2 OS cells 3 days after virus infection was evaluated by measuring the absorbance at 450 nm after treatment with a Cell Counting Kit-8 (Dojindo). Neutralizing antibody titer was determined as the maximum dilution at which 50% or mor of U-2 OS cells survived.

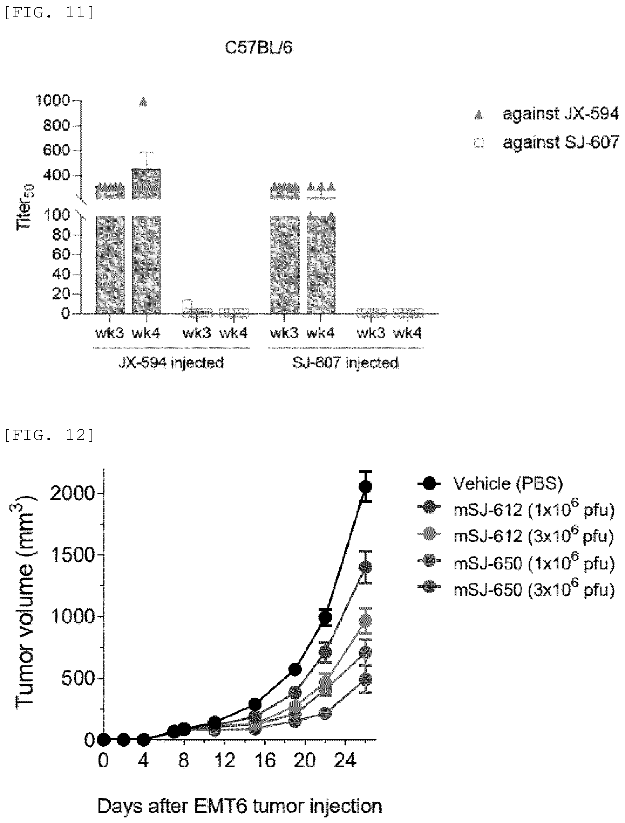

As a result, it was confirmed that antibodies against vaccinia virus present in the blood of BALB/c mice injected with JX-594 vaccinia virus effectively neutralized JX-594, but did not show neutralization to SJ-607 to a similar extent as those injected with SJ-607. On the other hand, antibodies against vaccinia virus present in the blood of mice injected with SJ-607 neutralized JX-594 vaccinia virus to a similar extent to those injected with JX-594 (FIG. 10). These results were also observed in C57BL/6 mice (FIG. 11). Therefore, it was confirmed that antibodies against vaccinia virus were formed in mice injected with SJ-607 vaccinia virus, but unlike JX-594, SJ-607 vaccinia virus could evade neutralization by antibodies against vaccinia virus.

### Example 4: Confirmation of antitumor efficacy upon repeated intravenous administration in breast cancer model

### 4.1. Confirmation of tumor growth inhibition efficacy

A syngeneic orthotopic breast cancer model was established by orthotopic transplantation of 2 x 10⁵ EMT-6 mouse triple-negative breast cancer cells into the mammary fat pad of naive, immunocompetent BALB/c mice. When the tumor size reached about 70-100 mm³, mSJ-650 (Preparation Example 3) that expresses CD55 on the viral IMV membrane, or a control virus mSJ-612 (Preparation Example 2) that does not express CD55, was repeatedly administered intravenously through the tail vein of the mouse twice a week at 3-4 day intervals, and the tumor growth inhibition efficacy of the viruses was compared. mSJ-650 and mSJ-612 were administered intravenously at low concentrations (1 x 10⁶ pfu) or high concentrations (3 x 10⁶ pfu), respectively.

Blood samples were collected from the orbital vein of the mouse at day 21 after the initial virus administration, left at room temperature for 30 minutes and then centrifuged at room temperature for 5 minutes to isolate serum. The isolated serum was heat-treated at 56°C for 30 minutes to inactivate complement in the serum, and then the formation of neutralizing antibodies against mSJ-612 or mSJ-650 was measured. The heat-treated serum was diluted 10- to 316,228-fold with cell culture medium and incubated with mSJ-612 or mSJ-650 for 2 hours to allow neutralizing antibodies against oncolytic vaccinia virus in the serum to neutralize the virus. U-2 OS human osteosarcoma cells seeded in 96-well plates were infected with mSJ-612 or mSJ-650 diluted with serum and incubated at 37°C for 3 days, and then the cell death effect was observed. The viability of U-2 OS cells 3 days after virus infection was evaluated by measuring the absorbance at 450 nm after treatment with a Cell Counting Kit-8 (Dojindo). Neutralizing antibody titer was determined as the maximum dilution at which more 50% or more of U-2 OS cells survived.

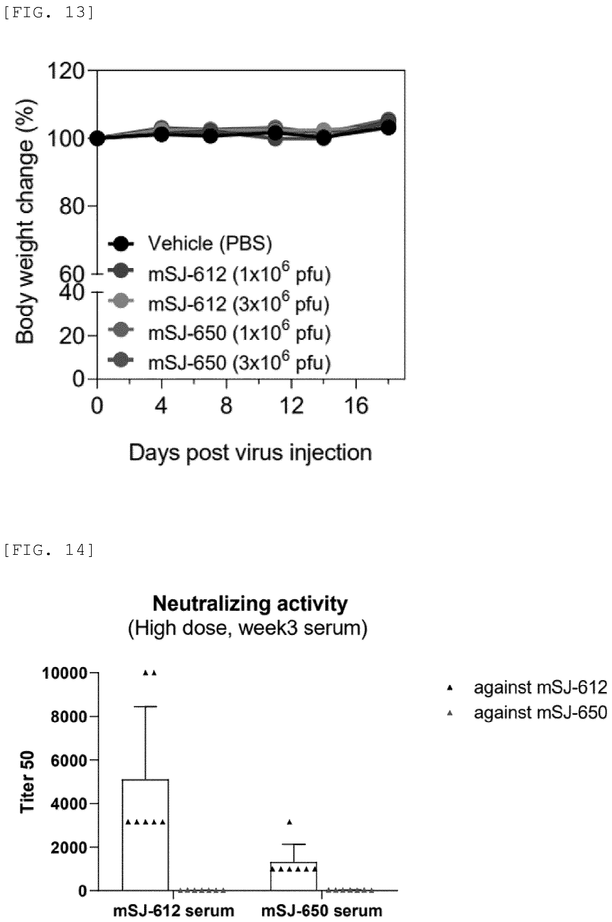

As a result, both mSJ-612 and mSJ-650 showed significant tumor growth inhibition efficacy compared to the negative control (PBS), and specifically, repeated intravenous administration of mSJ-650 inhibited tumor growth more significantly compared to repeated intravenous administration of mSJ-612 (FIG. 12). However, no significant difference was observed in changes in mouse body weight in all experimental groups (FIG. 13).

### 4.2. Confirmation of neutralization evasion ability

A EMT-6 mouse breast cancer model was repeatedly administered intravenously with an oncolytic virus at 3 x 10⁶ pfu twice a week at 3-4 day intervals. The amount of neutralizing antibodies produced against mSJ-612 or mSJ-650 virus in the blood of mouse was measured at day 21 after the initial virus administration.

As a result, as shown in FIG. 14, in the blood of mice repeatedly administered with mSJ-612 that does not express CD55, antibodies neutralizing mSJ-612 were formed in large quantities (mean Titer₅₀ ≒ 5,115.7), and the neutralizing ability against mSJ-650 in the same sample was about 389.2 times lower than that of mSJ-612 (mean Titer₅₀ ≒ 13.1). On the other hand, in the blood of mice repeatedly administered with mSJ-650 that expresses CD55, antibodies neutralizing mSJ-650 were formed to some extent (mean Titer₅₀ ≒ 25.7), but it was about 50.9 times lower than the neutralizing ability against mSJ-612 in the same sample (mean Titer50 ≒ 1,308.9).

These results demonstrated that sufficient antibodies against vaccinia virus were formed after injection of the vaccinia virus expressing CD55, but the virus expressing CD55 was able to evade the neutralizing effect of these antibodies.

### Example 5: Confirmation of neutralizing antibody resistance of mSJ-650 oncolytic virus upon repeated intravenous administration in breast cancer model

### 5.1. Direct injection of neutralizing antibody

To obtain serum containing neutralizing antibodies against oncolytic vaccinia virus, twenty naïve, immunocompetent BALB/c mice were administered 5 x 10⁶ pfu of oncolytic vaccinia virus mSJ-650 through the tail vein at 2-3 times at 1-week intervals. Whole blood of the mice was collected at day 28 after the initial virus administration. The collected blood samples were left at room temperature for 30 minutes, then centrifuged at room temperature for 5 minutes to isolate serum. All the isolated serum was pooled and aliquoted, and then frozen at -80°C until use. The amount of neutralizing antibodies present in the serum was measured in the same manner as in the experimental method in Example 4.

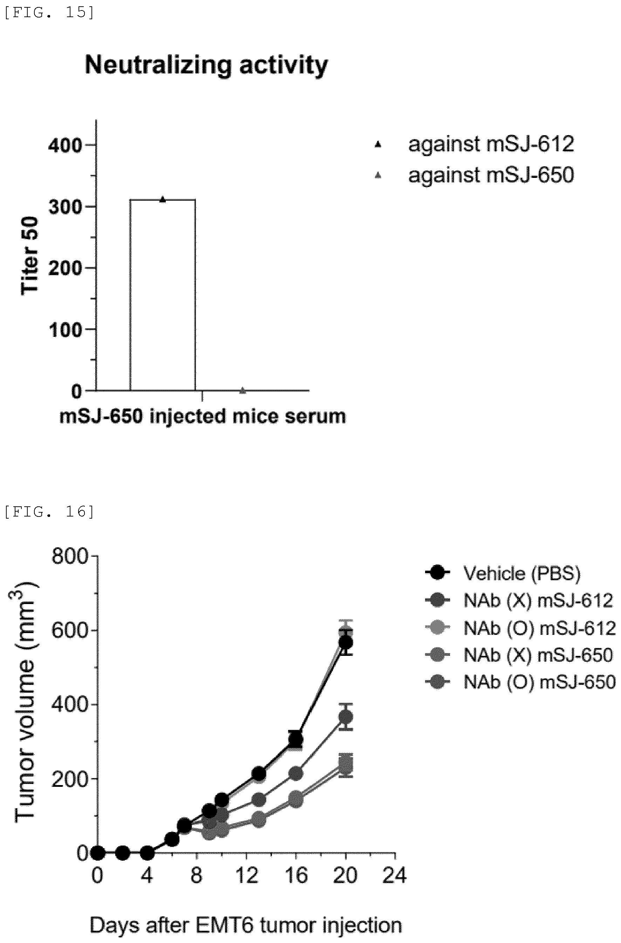

As a result, it was confirmed that the vaccinia viral antibodies present in the blood of mouse were able to neutralize mSJ-612 to a degree that was about 300 times higher than mSJ-650 (FIG. 15).

On the other hand, in the mouse breast cancer model established in Example 4.1, when the tumor size reached about 50-70 mm³, mSJ-650 that expresses CD55 on the viral IMV membrane, or the control virus mSJ-612 that does not express CD55, was repeatedly administered intravenously through the tail vein of the mouse at 3 × 10⁶ pfu twice a week at 3-4 day intervals. In addition, to verify the neutralization evasion ability of the mSJ-650 oncolytic virus, serum containing pre-obtained neutralizing antibodies was diluted in PBS and administered into the tail vein of the mouse 1 day before each administration of each treatment with the oncolytic virus, and the tumor growth inhibition efficacy was compared.

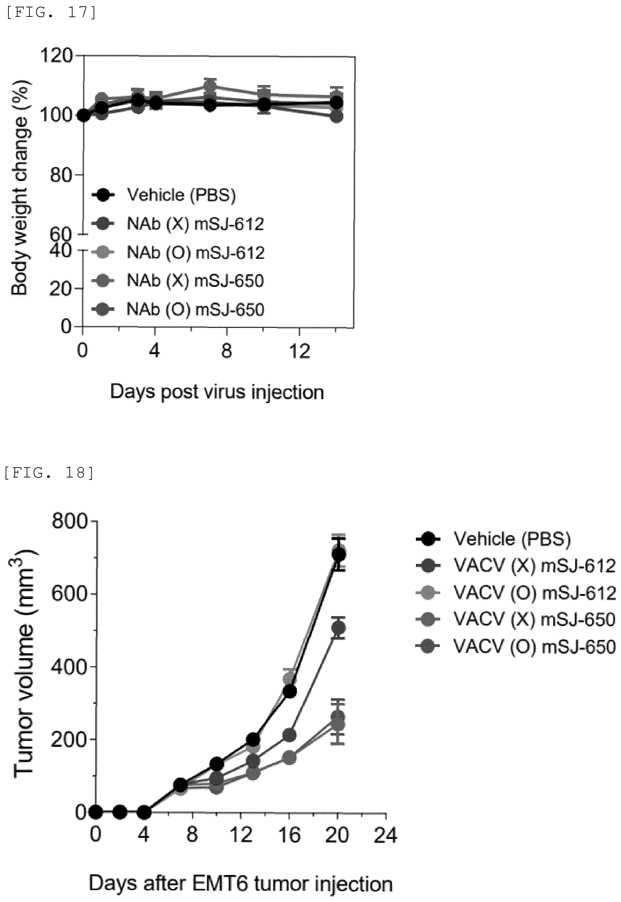

As a result, as shown in FIG. 16, in the case of mSJ-650 administration, significant tumor growth inhibition efficacy was observed compared to the negative control (PBS) from day 9 after tumor transplantation, regardless of whether serum containing antibodies against vaccinia virus was injected. On the other hand, it was confirmed that in the case of mSJ-612 administration, tumor growth inhibition was observed when serum containing antibodies against the virus was not injected, but the efficacy was lower compare to mSJ-650 administration, and when serum containing antibodies was injected, no tumor growth inhibition was observed at all. On the other hand, no significant difference was observed in changes in mouse body weight in all experimental groups (FIG. 17) .

### 5.2. Model for inducing neutralizing antibody formation by repeated virus injections

Two weeks before transplanting tumor cells into naive, immunocompetent BALB/c mice, mSJ-650 virus was administered intravenously through the tail vein at 3 x 10⁶ pfu twice at 1-week intervals to induce the formation of antibody against the virus. Two weeks after the initial virus administration, a syngeneic orthotopic breast cancer model was established by transplantation of 2 x 10⁵ EMT-6 mouse triple-negative breast cancer cells into the mammary fat pad of mice. When the tumor size reached about 50-70 mm³, mSJ-650 that expresses CD55 on the viral IMV membrane, or a control virus mSJ-612 that does not express CD55, was administered intravenously through the tail vein of mice at a dose of 3 x 10⁶ pfu twice a week at 3-4 days intervals. Through this, the tumor growth inhibition efficacy of the mSJ-650 oncolytic virus or mSJ-612 virus was compared between the group that was induced to form neutralizing antibodies against the vaccinia virus by administering the virus before tumor implantation and the group that did not receive the virus, and the ability of the mSJ-650 oncolytic virus to evade neutralizing responses was verified.

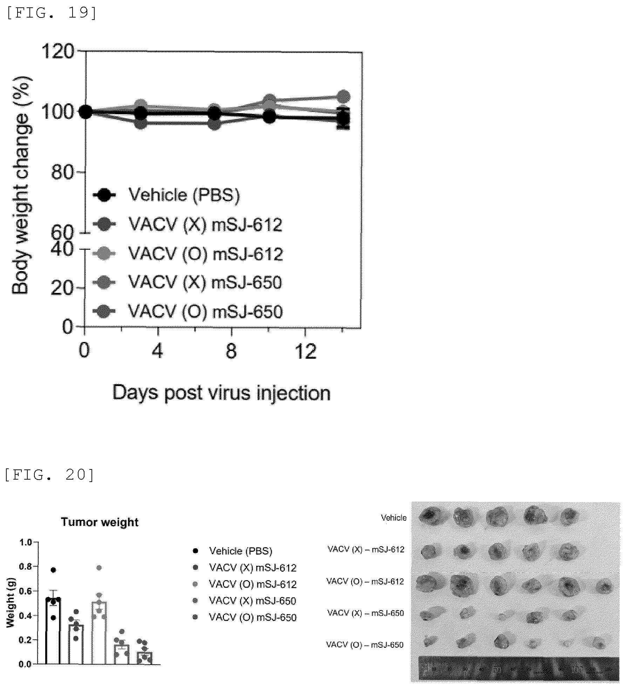

As a result, as shown in FIG. 18, mSJ-650 administration showed significant tumor growth inhibition efficacy regardless of whether the virus was pre-administered or not. On the other hand, mSJ-612 administration showed tumor growth inhibition efficacy but at a lower level than mSJ-650 administration when the virus was not pre-administered, and no tumor growth inhibition efficacy was observed at all when the virus was pre-administered. Meanwhile, no significant difference was observed in changes in mouse body weight in all experimental groups (FIG. 19).

In addition, after transplantation of EMT-6 mouse breast cancer cell, the mouse was sacrificed on day 21, and tumors were extracted and weighted. As a result, compared to the negative control (PBS) without virus injection, only the group that was pre-injected with vaccinia virus and administered mSJ-612 showed no difference in tumor size, and the remaining groups showed significant tumor size reduction efficacy. Even when compared to the group that was administered mSJ-612 without pre-injection of vaccinia virus, the group that was pre-injected with vaccinia virus and administered mSJ-650 showed significant tumor size suppression. In other words, it was proven that the mSJ-650 group showed tumor growth inhibition efficacy regardless of whether the virus was pre-administered or not, and unlike the mSJ-612 group, the anticancer efficacy of the virus was not reduced by neutralizing antibodies (FIG. 20).

From the above description, those skilled in the art to which the present disclosure pertains will understand that the present disclosure can be implemented in other specific forms without changing its technical idea or essential features. In this regard, it should be understood that the embodiments described above are exemplary in all respects and are not intended to be limiting. The scope of the present disclosure should be interpreted that all changes or modifications derived from the meaning and scope of patent claims to be described below rather than the detailed description above and their equivalent concepts are included in the scope of the present disclosure.

## Claims

1. An intravenously injectable composition for repeated administration, comprising, as an active ingredient, an oncolytic virus
in which: expression of a thymidine kinase (TK) gene is suppressed; and
a gene encoding complement regulatory protein CD55 fused with a transmembrane domain region of viral membrane protein to express the CD55 in an intracellular mature virion (IMV) membrane and a gene encoding granulocyte-macrophage colony-stimulating factor (GM-CSF) are inserted.

2. The intravenously injectable composition for repeated administration of claim 1, wherein the CD55 is composed of a sequence of SEQ ID NO:1.

3. The intravenously injectable composition for repeated administration of claim 1, wherein the membrane protein of the oncolytic virus is D8L, A16L, F9L, G9R, H3L, L1R, A9L, A13L, A21L, A28L, E10R, G3L, H2R, I2L, J5L, L5R, or O3L.

4. The intravenously injectable composition for repeated administration of claim 1, wherein the oncolytic virus is a vaccinia virus, an adenovirus, a herpes simplex virus, a retrovirus, a reovirus, a Newcastle disease virus, a Coxsackie virus, an enterovirus, or a herpesvirus.

5. The intravenously injectable composition for repeated administration of claim 4, wherein the vaccinia virus is Western Reserve (WR), New York Vaccinia Virus (NYVAC), Wyeth, LC16m8, Lister, Copenhagen, Tian Tan, USSR, TashKent, Evans, International Health Division-J (IHD-J), or International Health Division-White (IHD-W) strain.

6. The intravenously injectable composition for repeated administration of claim 1, wherein the composition evades neutralization by antibodies against the virus.

7. The intravenously injectable composition for repeated administration of claim 6, wherein the neutralization evasion is by expression of the CD55.

8. The intravenously injectable composition for repeated administration of claim 1, wherein the oncolytic virus has suppressed serine protease inhibitor-3 (SPI-3) gene expression.

9. A method for preventing or treating cancer, comprising: administering to a subject the composition of any one of claims 1 to 8.

10. Use of the composition of any one of claims 1 to 8 for preventing or treating cancer.

11. Use of the composition of any one of claims 1 to 8 for the manufacture of a medicament for preventing or treating cancer.
